# EUROPEAN PATENT APPLICATION

(11) **EP 2 101 120 A1**
(43) Date of publication of application: **16.09.2009**
(21) Application number: 09003383.8
(22) Date of filing: 09.03.2009
(51) Int. Cl.: F24F 13/02, F16L 9/00, A61L 2/23

(54) **Duct, panel and joining means for duct for conveying air with antimicrobial properties**

(30) Priority: 10.03.2008 IT MI20080393
(71) Applicant: Librizzi, Giuseppe, 24061 Albano S. Alessandro (BG) (IT)
(72) Inventor: Librizzi, Giuseppe, 24061 Albano S. Alessandro (BG) (IT)
(74) Representative: Nemni, Raffaelo

(57) **Abstract**

The invention falls into the field of ducts for conveying air for environmental conditioning, of panels for said ducts and of the relative joining means, with at least one internal face in metallic or plastic material, treated with a solution of silver ions and zeolite having an antimicrobial function.

## Description

### OBJECT OF THE INVENTION

The present invention relates to a duct for conveying air with a concentration of pathogenic micro-organisms in the internal air of said duct of below 49.25 ± 4 CFU/m³, gram positive 8± 3 CFU/m³. The present invention relates also to panels and joining means for making said duct.

Said duct is equipped with at least one surface on which silver ions are present. Said surface faces the inside of the duct in which the air is conveyed. The duct which is the subject of the present invention can be realised with panels which have silver ions and preferably zeolite on at least one surface. Furthermore the subject of the present invention are joining means for panels and portions of ducts which have silver ions and preferably zeolite on at least one surface.

### STATE OF THE ART

The walls of premises are made in various materials and finished with substantially synthetic paints. The walls of premises can also be made of various materials with a synthetic covering. In the case of premises used for activities which need to have hygienically specified air quality, the walls of the premises are made of easily cleanable materials, such as for example polyphenolic compounds. Said premises could for example be operating theatres, hospital premises, plants for producing food products or plants for producing semiconductors.

To maintain the level of air quality in said premises, the walls are subjected to frequent washing, forcing a stoppage of activity in said premises. Frequent washing, however, increases the risk that it may not be possible to restore the premises to the maximum levels of hygiene specified.

One of the factors which play a part in lowering the level of hygienic standards in premises served by air conditioning is the proliferation of micro-organisms inside the ducts which make up the air conditioning systems, particularly pathogenic micro-organisms. Known ducts are made with panels or conduits in metallic material. Said known ducts constitute a favourable environment for the proliferation of micro-organisms harmful to human beens because of the moisture level created in said ducts, which is favourable as we have said to the proliferation of micro-organisms which are naturally present in the air. To reduce the introduction into the premises concerned of micro-organisms harmful to human beens, filters are currently used for mechanically arresting the passage of air charged with micro-organisms present in the duct into the premises concerned.

### DISCLOSURE OF THE INVENTION

An object of the present invention is to create a duct which overcomes the disadvantages of known ducts and is capable of improving the quality of the air, by reducing the charge of pathogenic micro-organisms present in the air in the premises concerned. An object of the present invention is also to create a duct composed of panels capable of introducing air into the premises served by said duct, with a charge of pathogenic micro-organisms lower than that of known ducts. These objects are achieved through the use of silver ions applied to the inner surface of the duct in which the air concerned is present. Said ducts are preferably made with panels with thermally insulating characteristics, having silver ions on at least one surface of each panel making up the duct, positioned inside said duct in which the air concerned is contained. The use of said panel is preferred because the temperature variation inside the duct is lower than with ducts made of other materials, enabling the antimicrobial effect of the silver ions to be greater. To improve the antimicrobial effect of the ducts which are the subject of the present invention, joining means are preferably used for said panels and for portions of ducting, which have silver ions on at least one surface of said joining means. The surface of said joining means on which silver ions are present is positioned in such a way that it faces the inside of said duct containing the air concerned, so that there are silver ions present over the entire inner surface of the duct which is the subject of the present invention.

The micro-organisms usually contained in normal air are destroyed by contact thanks to the properties of said silver ions, advantageously reducing the presence of pathogenic micro-organisms present in the air passing through said ducts.

The presence of said silver ions can be achieved by applying to the entire surface over which the air passes a solution of silver ions and finishing paint for said surface, with a minimum concentration of silver ions of 0.5% by weight of said solution containing silver ions applied to said surface. Zeolite is preferably added to said solution to slow the release of the silver ions, thus obtaining a duration of years for the antimicrobial action of the silver ions. Said presence of silver ions on the mentioned surfaces can be achieved on the basis of the common knowledge of a person skilled in the art, without departing from the protective scope of the claims below.

Said duct surfaces are preferably made of metallic or plastic material or clad with such materials, on which silver ions are present in the minimum concentration quoted above. Said materials are preferred because their structure responds advantageously to the function of maintaining the silver ions on the surface so that they can perform their antimicrobial function.

In the present description, reference has been made to silver ions present on the inner surfaces. It is evident that the procedures through which the presence of silver ions on said surfaces may be achieved, may vary on the basis of the knowledge of a person skilled in the art, such as for example using sheets or films to which solutions containing silver ions have been applied, which are subsequently positioned on the inner surfaces of the duct or of the panels. Said procedures obviously do not depart from the protective scope of the claims stated below.

### EXPERIMENTAL SECTION

**Example 1** - For an evaluation of the quality of air in a duct for conveying air treated with silver ions, an assessment was made of the bacterial content present at the outlet of said air from said duct with respect to the bacterial content present at the outlet of said air from a duct made of galvanised steel sheet.

An assessment was made in minimum conditions of the efficiency of a duct having its internal surface treated with silver ions, in particular with a minimum concentration of silver ions of 0.5% by weight of the finishing paint applied over the internal surface of the duct. A duct made of conventional metallic material (galvanised steel sheet) was used as a conduit for comparison. The two ducts were kept ventilated, humidified, at a constant temperature by means of an identical heat source, simulating the conditions present inside an air conditioning duct. To simulate real conditions of use, the duct was fitted with a timeswitch, set to provide cycles of twelve hours, of which seven were working hours and five were rest hours. The system operated uninterruptedly for forty-eight days. The microclimate inside the ducts in the system was recorded throughout the period of operation of the system by two dataloggers (one for each outlet duct), to verify that the temperature and humidity inside the ducts was maintained within acceptable limits for the growth of micro-organisms. The antimicrobial effectiveness of the duct treated with silver ions compared with the galvanised steel duct was evaluated by sampling the surface. The air was sampled at the inlet and the outlet of the ducts by an SAS Microflow 6o sampler, fitted with plates containing a cultural medium suitable for the growth of micro-organisms (PSA).

The object of the experiment was to evaluate the antimicrobial efficacy of the surface treated with silver ions on the air quality at the outlet of said duct treated with silver ions, in comparison with the air at the outlet of the galvanised steel duct, both with and without filters at the outlets from the ducts. The biocontamination of the air leaving the duct treated with silver ions proved to be lower on a percentage basis than the air leaving the galvanised steel duct, both with and without exit filters.

In the presence of filters, the percentage reduction in the microbial count for the duct treated with silver ions compared with the galvanised steel duct was 46.9% (significance level 0.054), while in the absence of filters the reduction was 28.4% (significance level 0.353). The experimental test also demonstrated that in the presence of filters the percentage reduction in non spore-forming gram positive bacteria was found to be 54.4%, statistically significant (significance level 0.023), while in the absence of filters the percentage reduction in gram positive bacteria was found to be 54.8% (significance level 0.305).

The total microbial count level is due for the most part to the high presence of non-pathogenic spore-forming bacteria (not hazardous to human health), over which silver ions have a recognised lower antimicrobial efficacy. The percentage of total microbial count of micro-organisms for the duct treated with silver ions was lower by 49.25 ± 4 CFU/m³, gram positive 8 ± 3 CFU/m³.

### DESCRIPTION OF THE DRAWINGS

Figure 1 shows a perspective view of the duct which is the object of the present invention;
Figure 2 shows a perspective view of the panel which is the object of the present invention;
Figure 3 shows a perspective view of a joining means which is the object of the present invention.

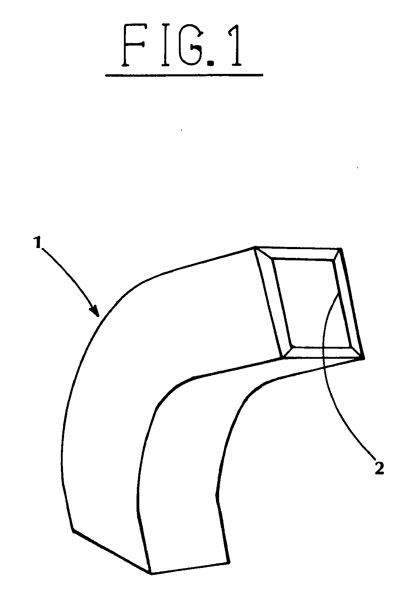

With reference to Figure 1, no. (1) indicates the duct which is the object of the present invention.

Said duct (1) has an internal surface (2) on which silver ions are present. The presence of said silver ions can be achieved by applying to the entire surface (2) a solution of silver ions and finishing paint for said surface (2), with a minimum, preferred, concentration of silver ions of 0.5% by weight of said solution containing said silver ions applied to said surface. Said solution preferably has zeolite added to it to slow the release of the above-mentioned silver ions. Said presence of silver ions on the surface (2) can also be achieved on the basis of the common knowledge of a person skilled in the art without departing from the protective scope of the claims below.

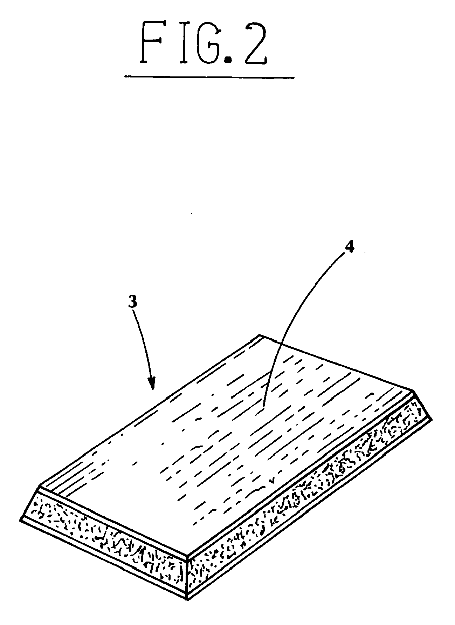

In figure 2, no. (3) indicates a panel with a surface (4) positioned in such a way as to be inside the duct on which silver ions are present, with the air in question passing through it. In this case, too, the presence of said silver ions can be achieved with the same procedures as were indicated in relation to the duct (1). The minimum, preferred, concentration of silver ions in this case too is 0.5% by weight of said solution containing silver ions applied to said surface (4); in this case, too, zeolite is preferably added to slow the release of the mentioned silver ions. Said presence of silver ions on the surface (4) can also be achieved on the basis of the common knowledge of a person skilled in the art without departing from the protective scope of the claims below.

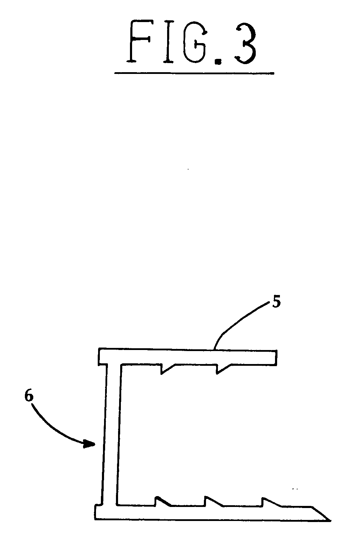

In figure 3, no. (6) indicates a joining means with a surface (5) positioned in such a way as to be inside the duct on which silver ions are present, with the air in question passing through it. In this case, too, the presence of said silver ions can be achieved with the same procedures as were indicated in relation to the duct (1). The minimum, preferred, concentration of silver ions in this case too is 0.5% by weight of said solution containing silver ions applied to said surface (5); in this case, too, zeolite is preferably added to slow the release of the mentioned silver ions. Said presence of silver ions on the surface (5) can also be achieved on the basis of the common knowledge of a person skilled in the art without departing from the protective scope of the claims below.

### MODE FOR CARRYING OUT THE PRESENT INVENTION

The present invention can be used for all environments served by air conditioning systems, in particular for those environments in which it is necessary to have a specified air quality. By way of non-exhaustive example, the following are indicated as environments in which a particular air quality is required: the air in operating theatres, industries in which food products are prepared, industries producing semiconductors, hospitals, hotels, hotel bedrooms, schools, offices, lecture halls, rest homes, restaurants, theatres and all places with public access. These types of ducts can also be advantageously used in motor vehicle air conditioning systems.

The present invention, therefore, makes it possible to have in any environment a better air quality as regards the presence of pathogenic micro-organisms than environments served by known air conditioning systems.

## Claims

1. Duct (1) for conveying air having in the internal air of said duct (1) a total concentration of pathogenic micro-organisms of below 49.25 ± 4 CFU/m³, gram positive 8± 3 CFU/m³ **characterised in that** silver ions are present on at least one surface (2) of the duct (1);

2. Duct (1) according to claim 1, **characterised in that** the surface (2) is made of metallic or plastic material;

3. Duct (1) according to claim 1, **characterised in that** the minimum concentration of silver ions on the surface (2) is of 0.5% by weight of the finishing paint solution or of other means applied to said surface (2) in which silver ions are present;

4. Duct (1) according to claim 1, **characterised in that** zeolite is present on the surface (2);

5. Duct (1) for conveying air having in the internal air of said duct (1) a total concentration of pathogenic micro-organisms of below 49.25 ± 4 CFU/m³, gram positive 8± 3 CFU/m³ **characterised in that** said duct (1) is made of one or more panels (3) having a surface (4) of panel (3) on which silver ions are present;

6. Duct (1) according to claim 5, **characterised in that** the surface (4) of panel (3) is made of metallic or plastic material;

7. Duct (1) according to claim 5, **characterised in that** the minimum concentration of silver ions on surfaces (4) of panel (3) is of 0.5% by weight of the finishing paint solution or of other means applied to said surface (4) in which silver ions are present;

8. Duct (1) according to claim 5, **characterised in that** zeolite is present on surface (4) ;

9. Duct (1) according to claim 1 or 5, **characterised in that** silver ions are present on at least one surface (5) of joining meanse (6) for panels (3) or ducts (1);

10. Duct (1) according to claim 9, **characterised in that** the surface (5) of the joining means (6) is made of metallic or plastic material;

11. Duct (1) according to claim 9, **characterised in that** the minimum concentration of silver ions on surfaces (5) of the joining means (6) is of 0.5% by weight of the finishing paint solution or of other means applied to said surface (6) in which silver ions are present;

12. Duct (1) according to claim 9, **characterised in that** zeolite is present on the surface (4).
